(19) Europäisches Patentamt
European Patent Office
Office européen des brevets

(11) Veröffentlichungsnummer: **0 213 295 B1**

(12) **EUROPÄISCHE PATENTSCHRIFT**

(45) Veröffentlichungstag der Patentschrift: **10.04.91**

(51) Int. Cl.5: **C07D 513/04**, A61K 31/425, //(C07D513/04,333:00,275:00)

(21) Anmeldenummer: **86108195.8**

(22) Anmeldetag: **16.06.86**

(54) **Neue Thieno-1,2-thiazolderivate, Verfahren zu ihrer Herstellung und diese enthaltende pharmazeutische Präparate.**

(30) Priorität: **04.07.85 AT 1988/85**

(43) Veröffentlichungstag der Anmeldung:
**11.03.87 Patentblatt 87/11**

(45) Bekanntmachung des Hinweises auf die Patenterteilung:
**10.04.91 Patentblatt 91/15**

(84) Benannte Vertragsstaaten:
**AT BE CH DE FR GB IT LI LU NL SE**

(56) Entgegenhaltungen:
**EP-A- 0 110 219**
**DE-B- 2 534 689**
**GB-A- 2 007 227**

(73) Patentinhaber: **Hafslund Nycomed Pharma Aktiengesellschaft**
**St. Peter-Strasse 25**
**A-4021 Linz(AT)**

(72) Erfinder: **Binder, Dieter, Dr.**
**Sieveringerstrasse 207**
**A-1190 Wien(AT)**

(74) Vertreter: **Kunz, Ekkehard, Dr.**
**Chemie Holding AG Patentwesen St. Peter-Strasse 25**
**A-4021 Linz(AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft neue Thieno-1,2-thiazolderivate der allgemeinen Formel

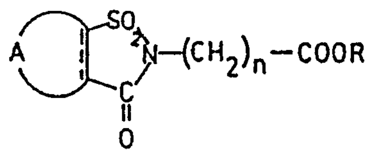

I

worin

A mit den beiden Kohlenstoffatomen des Thiazolrings eine Gruppe der Formel

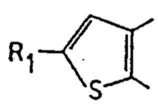

IIa

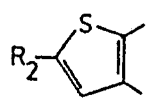

IIb

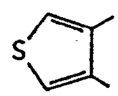

IIc

bildet und die gestrichelte Linie die in den Thieno Strukturen der Formeln IIa und IIb vorliegende Doppelbindung anzeigt,

n eine ganze Zahl 2,

R Wasserstoff, Ethyl oder 1,1-Dimethylethyl,

$R_1$ Wasserstoff und

$R_2$ Wasserstoff oder Chlor bedeuten, mit dem Proviso, daß, wenn $R_2$ Chlor bedeutet, R nur die Bedeutung von Wasserstoff oder 1,1-Dimethylethyl haben kann.

Die Thieno-1,2-thiazolderivate der allgemeinen Formel I werden erfindungsgemäß nach an sich bekannten Methoden, vorzugsweise dadurch hergestellt, daß man

a) eine Verbindung der allgemeinen Formel

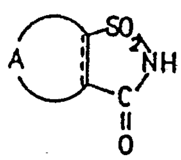

III

worin

A die in Formel I genannte Bedeutung besitzt, in ihr Natriumsalz verwandelt und dieses mit einer Verbindung der allgemeinen Formel

$$X-(CH_2)_n-COOR \qquad IV$$

worin

n und R die in Formel I angegebene Bedeutung haben und

X für Chlor oder Brom steht, umsetzt, und

b) gegebenenfalls eine so erhaltene Verbindung der allgemeinen Formel I, worin R 1,1-Dimethylethyl bedeutet, durch Erhitzen in Dimethylformamid, Dimethylsulfoxid oder Dimethylacetamid in Gegenwart von katalytischen Mengen p-Toluolsulfonsäure, Benzolsulfonsäure oder Methansulfonsäure in eine Verbindung der allgemeinen Formel I umwandelt, worin R Nasserstoff bedeutet.

Die Herstellung der wasserfreien Natriumsalze von Verbindungen der allgemeinen Formel III kann auf übliche Art und Weise geschehen, indem man beispielsweise eine Verbindung der Formel III mit berechneten Mengen wäßriger Natriumhydroxidlösung, oder mit der berechneten Menge alkoholischer Natriumalkoholatlösung, vorzugsweise einer alkoholischen Natriummethylat- oder Natriumethylatlösung, behandelt und durch Eindampfen der erhaltenen Lösung die Natriumsalze im Trockenrückstand gewinnt.
Auf besonders einfache und vorteilhafte Weise kann man die Natriumsalze für das erfindungsgemäße Verfahren auch in situ herstellen, indem man eine Lösung der Verbindung der allgemeinen Formel III in einem aprotischen polaren Lösungsmittel, welches auch bei der nachfolgenden Umsetzung des Natriumsalzes mit einer Verbindung der allgemeinen Formel IV Verwendung finden kann, beispielsweise in Dimetlylformamid, Dimethylsulfoxid oder Dimethylacetamid, mit der berechneten Menge Natriumhydrid, beispielsweise einer NatriumhydridSuspension in Weißöl, versetzt.

Die Umsetzung der Natriumsalze von Verbindungen der allgemeinen Formel III mit den Verbindungen der allgemeinen Formel IV nach Verfahrensschritt a) kann in An- oder Abwesenheit eines reaktionsinerten Lösungsmittels erfolgen. Vorteilhafterweise wird die Umsetzung in einem polaren, aprotischen Lösungsmittel, in dem die Natriumsalze gut löslich sind, durchgeführt, wobei bevorzugt die oben bei der Herstellung der Natriumsalze genannten Lösungsmittel in Frage kommen. Die Umsetzung erfolgt vorzugsweise durch Erwärmen, wobei Temperaturen von 80 bis 140° C mit einem Temperaturoptimum bei etwa 100° C besonders bevorzugt sind. In Abhängigkeit von der Reaktionstemperatur und der Reaktivität der jeweils eingesetzten Reaktionspartner, insbesondere von der Natur der Abgangsgruppe X, beträgt die Reaktionszeit zwischen einigen Stunden und mehreren Tagen.
Bei dieser Umsetzung erhaltene tert. Butylester der allgemeinen Formel I, in denen R einen 1,1-Dimethylethylrest darstellt, können, falls erwünscht, nach Verfahrensschritt b) in die freien Säuren der allgemeinen Formel I, worin R Wasserstoff bedeutet, verwandelt werden. Die Abspaltung der 1,1-Dimethylethyl-Gruppe in Form von Isobutylen unter Bildung der freien Carboxylgruppe wird dabei zweckmäßigerweise so durchgeführt, daß man die Ausgangsverbindung der allgemeinen Formel I (R = 1,1-Dimethylethyl) in einem aprutischen, reaktionsinerten Lösungsmittel, vorzugsweise einem aromatischen Kohlenwasserstoff, wie Benzol, Toluol oder Xylol löst und in Gegenwart von katalytischen Mengen einer starken Säure erhitzt. Bevorzugt werden für diesen Zweck starke Säuren, die in den genannten Lösungsmitteln teilweise oder zur Gänze löslich sind, beispielsweise Benzolsulfonsäure, p-Toluolsulfonsäure, Methansulfonsäure und dergleichen, verwendet. Die Reaktionstemperatur, bei der die Abspaltung der 1,1-Dimethylethyl-Gruppe durchgeführt wird, kann in einem weiten Temperaturbereich oberhalb Raumtemperatur nahezu beliebig gewählt werden, wobei sich die Rückflußtemperatur des Reaktionsgemisches als besonders vorteilhaft erwiesen hat.

Die Reaktionszeit hängt von der Reaktionstemperatur ab und beträgt beispielsweise bei Rückflußerbitzen des Reaktionsgemisches in Toluol etwa 90 Minuten.

Die Ausgangsverbindungen der allgemeinen Formel IV sind aus der Literatur bekannt. Die für das erfindungsgemäße Verfahren verwendeten Ausgangsverbindungen der allgemeinen Formel III sind entweder ebenfalls literaturbekannt (DF-OS-2 534 689, DE-OS-2 839 266 und DE-OS-2 749 640) oder können ausgehend von bekannten Produkten in an sich bekannter Weise hergestellt werden. Insbesondere können sie gemäß dem folgenden Reaktionsschema nach üblichen, jedem Fachmann geläufigen chemischen Arbeitsmethoden synthetisiert werden.
Die im Reaktionsschema angegebenen Startverbindungen der Formeln V und VII sind literaturbeannt (DE-OS-2 537 070, DF-OS-2 835 760, DE-OS-2 838 85) und EP-A-103 142). Falls nichts anderes angegeben ist, haben im folgenden Reaktionsschema A, $R_1$ und $R_2$ die bei der Formel I angegebene Bedeutung.

## Reaktionsschema

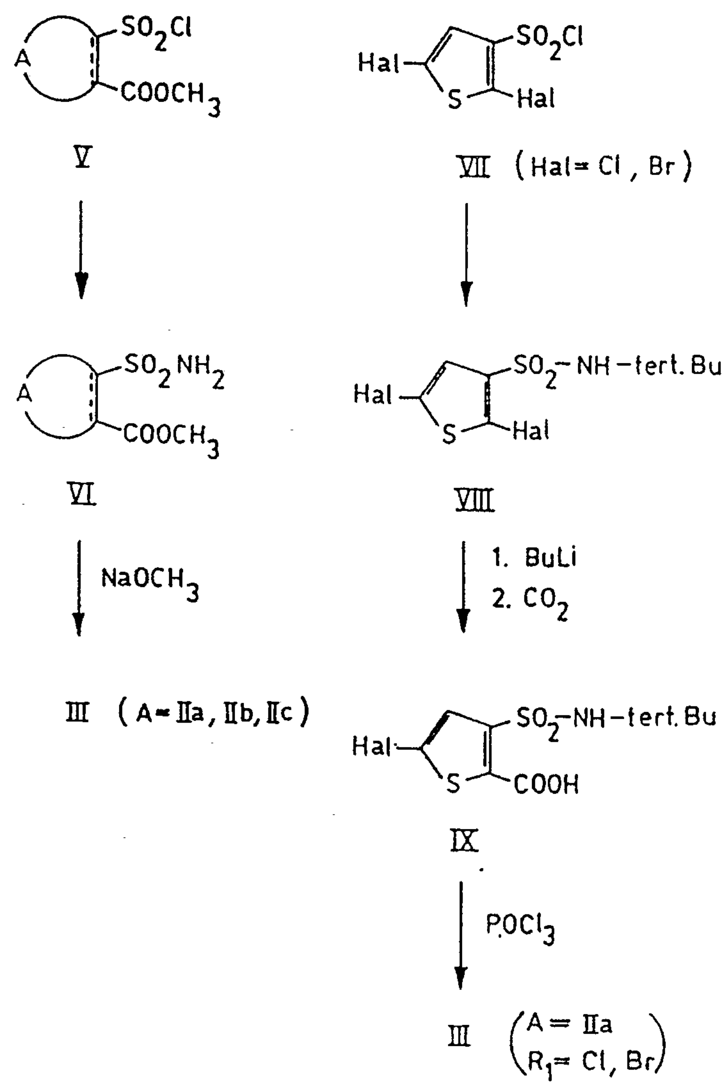


V

VII (Hal= Cl, Br)

VI

VIII

III (A= IIa, IIb, IIc)

IX

III (A = IIa, $R_1$= Cl, Br)

Die Veibindungen der allgemeinen Formel I zeigen im Tierversuch wertvolle pharmakologische Eigenschaften. Insbesondere bewirken sie eine starke Senkung der Cholesterin-und Triglycerid-Blutspiegelwerte und künnen aufgrund dieser lipidsenkenden Eigenschaften beispielsweise zur Behandlung und Prophylaxe von Erkrankungen, die durch einen erhöhten Cholesterin- und/oder Triglyceridspiegel im Blut hervurgerufen werden, in der Humanmedizin eingesetzt werden. Solche Krankheiten sind in erster Linie kardiovaskuläre Störungen, u.a. Thrombose, Arteriosklerose, Myokardinfarkt und Angina pectoris. Diese Wirkungen können durch in vitro Versuche oder in vivo-Tierversuche, vorzugsweise an Säugetieren, z.B. Meerschweinchen, Mäusen, Ratten, Katzen, Hunden oder Affen nachgewiesen werden. Die genannten Verbindungen können ihnen enteral oder parenteral, in Hinblick auf die humane Applikation insbesondere auch oral, verabreicht werden.

Zur Untersuchung der lipidsenkenden Eigenschaften von Verbindungen der allgemeinen Formel I wird u.a. folgende Testmethode verwendet:

Die Testsubstanzen werden in jeweils frisch bereiteter 1 %-iger Carboxymethylcellulose suspendiert und männlichen Mäusen (Stamm: OF 1, Swiss, SPF; Gewicht bei Versuchsbeginn: ca. 25 g), die freien Zugang zu einer Standarddiät und Trinkwasser haben, über einen Zeitraum von 14 Tagen einmal täglich in konstanten Dosen von 20 mg/kg mit dem einheitlichen Applikationsvolumen von 10 ml/kg intraperitoneal

verabreicht. Die Kontrollgruppe erhält nur 10 ml/kg 1%-ige Carboxymethylcellulose.
4 Stunden nach der letzten Applikation der Testsubstanzen bzw. der Carboxymethylcellulose in der Kontrollgruppe werden die Tiere durch Entbluten über die Aorta carotis getötet. Zur Bestimmung der Lipid-Blutspiegelwerte wird EDTA (Ethylendiamintetraessigsäure)-Plasma der Versuchstiere gewonnen.

Analysenmethoden:

Cholesterin wird sowohl nach der klassischen Methode (Farbtest nach Liebermann-Burchard; (Zbl. Pharm, 124 (7), 396 ff, 1985) als auch nach einer vollenzymatischen Methode (Celichrom Cholesterin; Testsystem: Firma Chemie Linz AG, Diagnostica; Linz, Österreich) bestimmt. Die Triglyceride werden vollenzymatisch (Triglyceride Jodonitratetrazoliumviolett; Testsystem: Firma Chemie Linz AG, Diagnostica; Linz, Österreich) bestimmt.
In diesem Test zeigen Verbindungen der allgemeinen Formel I stark lipidsenkende Eigenschaften. Beispielsweise bewirkt das 2,3-Dihydro-3-oxo-thieno(3,4-d)-1,2-thiazol-propionsäure-1-1-dioxid bei einer Dosierung von 20 mg/kg i.p. eine 9,2%-ige Senkung des Cholesterin- und eine 7,8%-ige Senkung des Triglycerid-Blutspegelwertes gegenüber der Kontrollgruppe.

Die Verbindungen der allgemeinen Formel I können als Heilmittel, z.B. in Form von pharmazeutischen Präparaten Verwendung finden, welche sie in Mischung mit einem für die enterale oder parenterale Applikation geeigneten pharmazeutischen, organischen oder anorganischen inerten Hilfs- und/oder Trägermaterial, wie z.B. pharmazeutisch unedenklichen Lösungsmitteln, Gelatine, Gummi arabicum, Milchzucker, Stärke, Magnesiumstearat, Talk, pflanzliche Öle, Polyalkylenglykole, Vaseline und dergleichen, enthalten.
Die pharmazeutischen Praparate können in fester Form, beispielsweise als Tabletten, Dragees, Suppositorien, Kapseln und dergleichen oder in flüssiger Form, beispielsweise als Lösungen, Suspensionen oder Emulsionen, vorliegen. Gegebenenfalls sind sie sterilisiert und enthalten Hilfsstoffe, wie Konservierungs-, Stabilisierungs- oder Emulgiermittel, Salze zur Veränderung des osmotischen Druckes und dergleichen.
Insbesondere können pharmazeutische Praparate die erfindungsgemäßen Verbindungen in Kombination mit anderen therapeutisch wertvollen Stoffen enthalten. Mit diesen können die erfindungsgemäßen Verbindungen zusammen mit den oben angegebenen Hilfs- und/oder Trägerstoffen zu Kombinationspräparaten formuliert werden.

Die folgenden Beispiele erläutern die Erfindung näher:

Beispiel 1:

10,0 g (0,053 Mol) Thieno(2,3-d)-1,2-thiazol-3 (2H)-on-1,1-dioxid werden in 100 ml trockenem Dimethylformamid suspendiert, mit 2,54 g (0,053 mol) einer mit Benzol gewaschenen 50 prozentigen Natriumhydridsuspension versetzt, bei 70° C unter Rühren (10 min) gelöst, auf 30° C abgekühlt, mit 12,16 g (0,058 mol) 3-Brompropionsäure-1,1-dimethylethylester versetzt und 3 Stunden auf 100° C erhitzt. Dann wird im Vakuum eingedampft, der Rückstand zwischen NaH-$CO_3$-ösung und $CH_2Cl_2$ verteilt, die organische Phase abgetrennt, mit $Na_2SO_4$ getrocknet und eingedampft. Der kristalline Rückstand, bestehend aus kann aus 1,1-Dimethylethyl-2,3-dihydro-3-oxo-thieno(2,3-d)-1,2-thiazol-propionat-1,1-dioxid, kann aus Diisopropylether umkristallisiert werden.

Ausbeute: 11 g (65,5 %)
Schmp. (Diisopropylether): 66-67° C farblose Kristalle

Beispiel 2:

10,0 g (0,053 Mol) Thieno(3,2-d)-1,2-thiazol-3(2H)-on-1,1-dioxid werden in 100 ml trockenem Dimethylsulfoxid suspendiert und in analoger Weise, wie in Beispiel 1 angegeben, 4 Stunden bei 95° C umgesetzt. Man erhält 67 % Ausbeute an 1,1-Dimethylethyl-2,3-dihydro-3-oxo-thieno(3,2-d)-1,2-thiazol-propionat-1,1-dioxid.

Schmp. (Diisopropylether): 72-74° C

Beispiel 3:

10 g (0,053 Mol) Thieno(3,4-d)-1,2-thiazol-3(2H)-on-1,1-dioxid werden in 100 ml trockenem Dimethylacetamid suspendiert und in analoger Weise, wie in Beispiel 1 angegeben, 3 Stunden bei einer Reaktionstemperatur von 110° C umgesetzt. Man erhält 62 % Ausbeute an 1,1-Dimethylethyl-2,3-dihydro-3-oxo-thieno(3,4-d)-1,2-thiazol-propionat-1,1-dioxid.

Schmp. (Diisopropylether): 94-95° C

Beispiel 4

10 g (0,045 Mol) 5-Chlor-thieno(3,2-d)-1,2-thiazol-3(2H)-on-1,1-dioxid werden in analoger Weise wie in Beispiel 1 angegeben, mit 10,03 g (0,048 Mol) 3-Brompropionsäure-1,1-dimethylester umgesetzt. Man erhält 60 % Ausbeute an 1,1-Dimethylethyl-5-chlor-2,3-dihydro-3-oxo-thieno(3,2-d)-1,2-thiazol-propionat-1,1-dioxid.

Schmp. (Diisopropylether): 115-116° C

Beispiel 5:

10 g (0,053 Mol) Thieno(2,3-d)-1,2-thiazol-3(2H)-on werden mit einer Lösung von 2,12 g (0,053 Mol) Natriumhydroxid in 150 ml Wasser versetzt und bis zum Erhalt einer nahezu rückstandsfreien Lösung gerührt. Von geringen Mengen an unlöslichen Anteilen wird abfiltriert und durch Verdampfen des Wassers im Vakuum der Trockenrückstand hergestellt. Das so erhaltene Alkalisalz wird fein pulverisiert und im Hochvakuum bis zur Gewichtskonstanz getrocknet. Dieses wird in 100 ml trockenem Dimethylformamid suspendiert, bei 80° C unter Rühren gelöst, auf 30° C abgekühlt, mit 7,92 g (0,058 Mol) 3-Chlorpropionsäureethylester versetzt und 4 Stunden 15 Minuten auf 100° C erhitzt.
Die Aufarbeitung des Reaktionsansatzes wird, wie in Beispiel 1 angegeben, durchgeführt. Man erhält in 65 %-iger Ausbeute Ethyl-2,3-dihydro-3-oxo-thieno(2,3-d)-1,2-thiazol-propionat-1,1-dioxid.

Schmp. (Diisopropylether): 62-63° C

Beispiel 6:

10 g (0,053 Mol) Thieno(3,2-d)-1,2-thiazol-3(2H)-on werden mit einer Lösung von 3,61 g (0,053 Mol) Natriumethylat in 120 ml trockenem Ethanol versetzt und bis zum Erhalt einer klaren Lösung gerührt. Durch Verdampfen des Lösungsmittels im Vakuum wird der Trockenrückstand hergestellt. Das hiebei erhaltene Natriumsalz wird fein pulverisiert, bis zur Gewichtskonstanz getrocknet, anschließend in 100 ml trockenem Dimethylformamid suspendiert, bei 80° C unter Rühren gelöst, auf 30° C abgekühlt und mit 10,49 g (0,058 Mol) 3-Brompropionsäureethylester versetzt. Zur Vervollständigung der Umsetzung wird noch 3 Stunden auf 100° C erhitzt.
Die Aufarbeitung des Reaktionsansatzes wird, wie in Beispiel 1 angegeben, durchgeführt. Man erhält in 60 %-iger Ausbeute Ethyl-2,3-dihydro-3-oxo-thieno (3,2-d)-1,2-thiazol-propionat-1,1-dioxid.

Schmp. (Diisopropylether): 95-96° C.

Beispiel 7:

10 g (0,053 Mol) Thieno(3,4-d)-1,2-thiazol-3(2H)-on-1,1-dioxid werden in 100 ml trockenem Dimethylacetamid suspendiert und in analoger Weise wie in Beispiel 1 angegeben mit Natriumhydrid behandelt und 2 Stunden 40 Minuten bei 110° C mit 10,49 g (0,058 Mol) 3-Brompropionsäureethylester umgesetzt. Man erhält 65% Ausbeute an Ethyl-2,3-dihydro-3-oxo-thieno(3,4-d)-1,2-thiazol-propionat-1,1-dioxid.

Schmp. (Diisopropylether): 105-107° C.

Beispiel 8:

10,8 g 1,1-Dimethylethyl-2,3-dihydro-3-oxo-thieno(2,3-d)-1,2-thiazol-propionat-1,1-dioxid werden in 110 ml trockenem Toluol gelöst, 113 mg p-Toluolsulfonsäure zugegeben und die Reaktionsmischung 90 Minuten rückflußerhitzt. Nach dem Erkalten werden die farblosen Kristalle, bestehend aus 2,3-Dihydro-3-oxo-thieno-(2,3-d)-1,2-thiazol-propionsäure-1,1-dioxid abgesaugt und aus Ethanol umkristallisiert.

Ausbeute: 79 %
Schmp.: 133-135° C.

Beispiel 9:

Bei zu Beispiel 8 analoger Umsetzung von 1,1-Dimethylethyl-2,3-dihydro-3-oxo- thieno(3,2-d)-1,2-thiazol-propionat-1,1-dioxid in 120 ml trockenem Benzol unter Rückfluß erhitzen werden 85 % Ausbeute an 2,3-Dihydro-3-oxo-thieno(3,2-d)-1,2thiazol-propionsäure-1,1-dioxid erhalten.

Schmp. (Wasser): 140-142° C

Beispiel 10:

10,8 g 1,1-Dimethylethyl-2,3-dihydro-3-oxo-thieno(3,4-d)-1,2-thiazol-propionat-1,1-dioxid werden in 100 ml trockenem Toluol gelöst, 110 mg Benzolsulfonsäure zugegeben und die Reaktionsmischung 95 Minuten am Rückfluß erhitzt. Man erhält in 78 %-iger Ausbeute 2,3-Dihydro-3-oxo-thieno(3,4-d)-1,2-thiazolpropions äure-1,1-dioxid.

Schmp. (Wasser): 173-176° C.

Beispiel 11:

8 g 1,1-Dimethylethyl-5-chlor-2,3-dihydro-3-oxo-thieno(3,2-d)-1,2-thiazol-propionat-1,1-dioxid werden in 90 ml trockenem Xylol gelöst, 95 mg Methansulfonsäure zugegeben und das Reaktionsgemisch unter Rühren für 105 Minuten auf 115° C erhitzt.
Man erhält in 82 %-iger Ausbeute 5-Chlor-2,3-dihydro-3-oxo-thieno(3,2-d)-1,2-thiazol-propionsäure-1,1-dioxid.

Schmp. (Ethanol): 195-197° C.

## Ansprüche

**1.** Thieno-i,2-thiazolderivate der allgemeinen Formel I

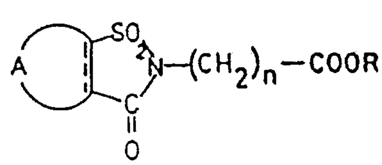

I

worin

A mit den beiden Kohlenstoffatomen des Thiazolrings eine Gruppe der Formel

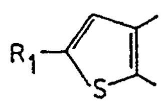 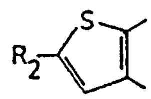 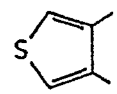

IIa IIb IIc

bildet und die gestrichelte Linie die in den Thieno-Strukturen der Formeln IIa und IIb vorliegende Doppelbindung anzeigt,
n eine ganze Zahl 2,
R Wasserstoff, Ethyl oder 1,1-Dimethylethyl,
$R_1$ Wasserstoff und
$R_2$ Wasserstoff oder Chlor beueuten, mit dem Proviso, daß, wenn $R_2$ Chlor bedeutet, R nur die Bedeutung von Wasserstoff oder 1,1-Dimethylethyl haben kann.

**2.** Verfahren zur Herstellung von Thieno-1,2-thiazolderivaten der in Anspruch 1 definierten allgemeinen Formel I, dadurch gekennzeichnet, daß man
a) eine Verbindung der allgemeinen Formel III

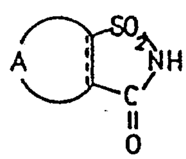

III

worin
A die in Formel I genannte Bedeutung besitzt, in ihr Natriumsalz verwanelt und dieses mit einer Verbindung der allgemeinen Formel

$$X-(CH_2)_n-COOR \qquad IV$$

worin
n und R die in Formel I angegebene Bedeutung haben und
X für Chlor oder Brom steht, umsetzt, und
b) gegebenenfalls eine so erhaltene Verbindung der allgemeinen Formel 1, worin R 1,1-Dimethylethyl bedeutet, durch Erhitzen in Dimethylformamid, Dimethylsulfoxid oder Dimethylacetamid in Gegenwart von katalytischen Mengen p-Toluolsulfonsäure, Benzolsulfonsäure oder Methansulfonsäure in eine Verbindung der allgemeinen Formel I umwandelt, worin R Wasserstoff bedeutet.

Patentansprüche für folgenden Vertragsstaat AT

**1.** Verfahren zur Herstellung von neuen Thieno-1,2-thiazolderivaten der allgemeinen Formel

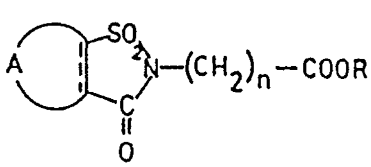

I

worin
A mit den beiden Kohlenstuffatomen des Thiazolrings eine Gruppe der Formel

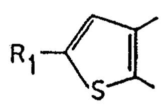
IIa

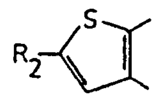
IIb

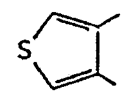
IIc

bildet und die gestrichelte Linie die in den Thieno-Strukturen der Formeln IIa und IIb vorliegende Doppelbindung anzeigt:
n eine ganze Zahl 2,
R Wasserstoff, Ethyl oder 1,1-Dimethylethyl,
$R_1$ Wasserstoff und
$R_2$ Wasserstoff oder Clor bedeuten, mit dem Proviso, daß, wenn $R_2$ Chlor bedeutet, R nur die Bedeutung von Wasserstoff oder 1,1-Dimethylethyl haben kann, dadurch gekennzeichnet, daß man

a) ein Verbindung der allgeneinen Formel

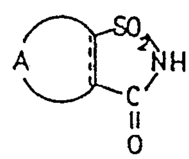
III

worin
A die oben genannte Bedeutung besitzt, in ihr Natriumsalz verwandelt und dieses mit einer Verbindung der allgemeinen Formel

$$X-(CH_2)_n-COOR \qquad IV$$

worin
n und R die oben angegebene Bedeutung haben und
X für Chlor oder Brom steht, umsetzt, und

b) gegebenenfalls eine so erhaltene Verbindung der allgemeinen Formel I, worin R 1,1-Dimethylethyl bedeutet, durch Erhitzen in Dimethylformamid, Dimethylsulfoxid oder Dimethylacetamid in Gegenwart von katalytischen Mengen von p-Toluolsulfonsäure, Benzolsulfonsäure oder Methansulfonsäure in eine Verbindung der allgemeinen Formel I umwandelt, worin R Wasserstoff bedeutet.

2. Verfahren nach Anspruch 1 dadurch gekennzeichnet, daß man die Abspaltung der 1,1-Dimethylethyl-Gruppe im Verfahrensschritt b) in Benzol, Toluol oder Xylol in Gegenwart von katalytischen Mengen p-Toluolsulfonsäure, Benzolsulfosäure oder Methansulfonsäure durchführt.

## Claims

1. Thieno-1,2-thiazole derivatives of the general formula I

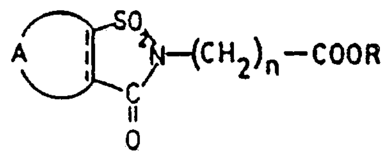 I

in which
A with the two carbon atoms of the thiazole ring forms a group of the formula

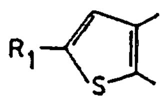 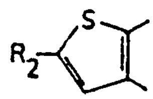 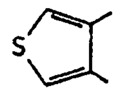

IIa IIb IIc

and the dashed line indicates the double bond present in the thieno structures of the formulae IIa and IIb,
n denotes an integer 2,
R denotes hydrogen, ethyl or 1,1-dimethylethyl,
$R_1$ denotes hydrogen and
$R_2$ denotes hydrogen or chlorine, with the proviso that, if $R_2$ denotes chlorine, R may only have the meaning hydrogen or 1,1-dimethylethyl.

**2.** Process for the preparation of thieno-1,2-thiazole derivatives of the general formula I defined in Claim 1, characterized in that
a) a compound of the general formula III

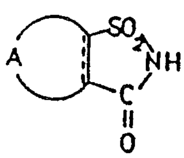 III

in which
A has the meaning mentioned in formula I, is converted into its sodium salt and this is reacted with a compound of the general formula

$$X-(CH_2)_n-COOR \quad IV$$

in which
n and R have the meaning indicated in formula I and
x represents chlorine or bromine, and
b) if desired, a compound of the general formula I thus obtained, in which R denotes 1,1-dimethylethyl, is converted into a compound of the general formula I in which R denotes hydrogen by heating in dimethylformamide, dimethyl sulphoxide or dimethylacetamide in the presence of catalytic amounts of p-toluenesulphonic acid, benzenesulphonic acid or methanesulphonic acid.

Claims for the following Contracting State : AT

1. Process for the preparation of novel thieno-1,2-thiazole derivatives of the general formula

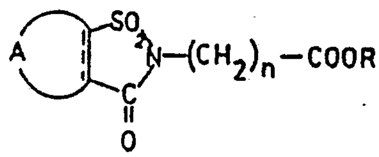

I

in which
A with the two carbon atoms of the thiazole ring forms a group of the formula

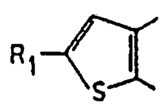

IIa

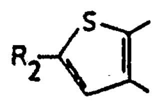

IIb

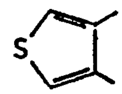

IIc

and the dashed line indicates the double bond present in the thieno structures of the formulae IIa and IIb,
n denotes an integer 2,
R denotes hydrogen, ethyl or 1,1-dimethylethyl,
R, denotes hydrogen and
$R_2$ denotes hydrogen or chlorine, with the proviso that, if $R_2$ denotes chlorine, R may only have the meaning hydrogen or 1,1-dimethylethyl, characterized in that

a) a compound of the general formula

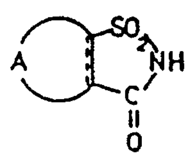

III

in which
A has the abovementioned meaning, is converted into its sodium salt and this is reacted with a compound of the general formula

$$X-(CH_2)_n-COOR \qquad IV$$

in which
n and R have the meaning indicated above and
x represents chlorine or bromine, and

b) if desired, a compound of the general formula I thus obtained, in which R denotes 1,1-dimethylethyl, is converted into a compound of the general formula I in which R denotes hydrogen by heating in dimethylformamide, dimethyl sulphoxide or dimethylacetamide in the presence of catalytic amounts of p-toluenesulphonic acid, benzene sulphonic acid or methanesulphonic acid.

2. Process according to Claim 1, characterized in that the elimination of the 1,1-dimethylethyl group in process step b) is carried out in benzene, toluene or xylene in the presence of catalytic amounts of p-toluenesulphonic acid, benzenesulphonic acid or methanesulphonic acid.

## Revendications

1. Dérivês thiéno-1,2-thiazole de formule générale I

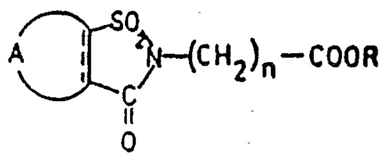

I

dans laquelle
A forme, avec les deux atomes de carbone du noyau thiazole, un groupe de formule

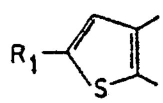

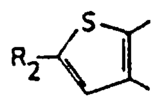

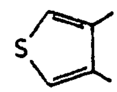

IIa IIb IIc

et le trait pointillé indique la double liaison présente dans les structures thiéno de formules IIa et IIb,
n représente un nombre entier égal à 2,
R représente un hydrogène, un groupe éthyle ou 1,1-diméthyléthyle,
$R_1$ représente un hydrogène et
$R_2$ représente un hydrogène ou un chlore, à condition que, si $R_2$ désigne un chlore, R ne puisse représenter qu'un hydrogène ou un groupe 1,1-diméthyléthyle.

2. Procédé de préparation de dérivés thiéno-1,2-thiazole de formule générale I définie dans la revendication 1, caractérisé en ce que
   a) l'on transforme un composé de formule générale III

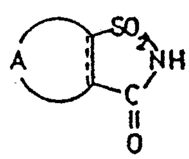

III

dans laquelle
A a la signification indiquée dans la formule I, en son sel de sodium et on le fait réagir avec un composé de formule générale X-$(CH_2)$n-COOR IV dans laquelle
n et R ont la signification indiquée dans la formule I et
x représente un chlore ou un brome, et
b) éventuellement, on transforme un composé de formule générale I, dans laquelle R représente un groupe 1,1-diméthyléthyle, ainsi obtenu en un composé de formule générale I, dans laquelle R représente un hydrogène, en le chauffant dans le diméthylformamide, le diméthylsulfoxyde ou le diméthylacétamide, en présence de quantités catalytiques d'acide p-toluènesulfonique, d'acide benzènesulfonique ou d'acide méthanesulfonique.

Revendications pour l'Etat contractant suivant: AT

1. Procédé de préparation de nouveaux dérivés thiéno1,2-thiazole de formule générale

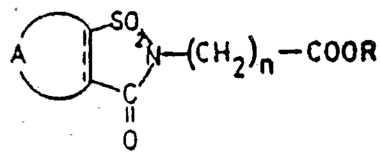

I

dans laquelle
A forme, avec les deux atomes de carbone du noyau thiazole, un groupe de formule

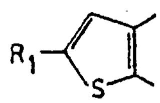

IIa

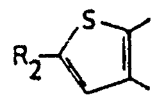

IIb

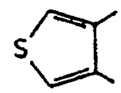

IIc

et le trait pointillé indique la double liaison présente dans les structures thiéno de formules IIa et IIb,
n représente un nombre entier égal à 2,
R représente un hydrogène, un groupe éthyle ou 1,1-diméthyléthyle,
$R_1$ représente un hydrogène et
$R_2$ représente un hydrogène ou un chlore, à condition que, si $R_2$ désigne un chlore, R ne puisse représenter qu'un hydrogène ou un groupe 1,1-diméthyléthyle, caractérisé en ce que

a) l'on transforme un composé de formule générale

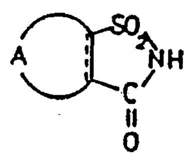

III

dans laquelle
A a la signification indiquée ci-dessus, en son sel de sodium et on le fait réagir avec un composé de formule générale

$$X-(CH_2)_n-COOR \qquad IV$$

dans laquelle
n et R ont la signification indiquée ci-dessus et
x représente un chlore ou un brome, et

b) éventuellement, on transforme un composé de formule générale I, dans laquelle R représente un groupe 1,1-diméthyléthyle, ainsi obtenu en un composé de formule générale I, dans laquelle R représente un hydrogène, en le chauffant dans le diméthylformamide, le diméthylsulfoxyde ou le diméthylacétamide, en présence de quantités catalytiques d'acide p-toluènesulfonique, d'acide benzènesulfonique ou d'acide méthanesulfonique.

2. Procédé selon la revendication 1, caractérisé en ce que l'on effectue l'élimination du groupe 1,1-diméthyléthyle, dans l'étape b) du procédé, dans le benzène, le toluène ou le xylène, en présence de quantités catalytiques d'acide p-toluènesulfonique, d'acide benzènesulfonique ou d'acide méthanesulfonique.